# EUROPEAN PATENT SPECIFICATION

(11) **EP 0 507 001 B1**
(45) Date of publication and mention of the grant of the patent: **17.09.1997**
(21) Application number: 91119129.4
(22) Date of filing: 11.11.1991
(51) Int. Cl.: C07D 295/10

(54) **A process for the preparation of nepinalone**
Verfahren zur Herstellung von Nepinolon
Procédé pour la préparation du népinalone

(30) Priority: 05.04.1991 IT MI910952
(43) Date of publication of application: 07.10.1992
(73) Proprietor: BIOINDUSTRIA FARMACEUTICI S.p.A., 04100 Latina (IT)
(72) Inventor: Tenconi, Franco, I-15067 Novi Ligure (AL) (IT); Furfaro, Silvana, I-15067 Novi Ligure (AL) (IT)
(74) Representative: Minoja, Fabrizio

(56) References cited:
- GB-A- 1 140 990
- ORGANIC PREPARATIONS AND PROCEDURES INTERNATIONAL, vol. 16, no. 3-4, 1984, pages 294 - 297 D.C. HUNDEN 'An improved synthesis of beta-tetralone'
- JOURNAL OF ORGANIC CHEMISTRY. vol. 26, November 1961, WASHINGTON DC US pages 4232 - 4235 J.H. BURCKHALTER E.A. 'Ethylene and Phenacetyl Chloride in the Friedel-Crafts Reaction. Novel Syntheses of 2-Tetralones and Benzofuranones'

## Description

The present invention relates to a process for the preparation of Nepinalone of formula (I):

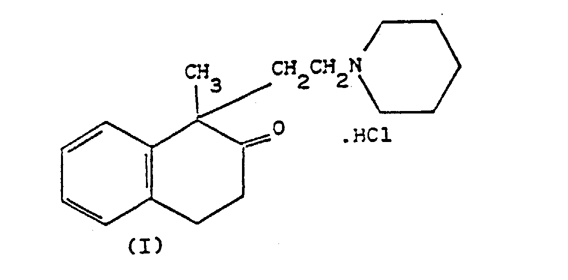

Nepinalone is a compound having antituxive activity, it is disclosed in GB 1140990. In said patent, Nepinalone was prepared by reacting 1-methyl-2-tetralone with 1-N-(2-chloroethyl)piperidine, in the presence of NaH and using toluene as the solvent. 1-Alkyl-2-tetralone was prepared, through enamine, by reacting 2-tetralone with pyrrolidine, methylating with methyl iodide and subsequently restoring the keto group by means of acid hydrolysis. 2-Tetralone, in its turn, was prepared by reducing 2-methoxynaphthalene with sodium in ethanol and subsequent hydrolysis (Org. Syntheses vol. 32, p. 97).

An alternative preparation of 1-alkyl-2-tetralones was described by S.D.Mekhtiev et al. (Azerb. Khim. 24, 1963 (5), 17-22) and by A. Rosowsky et al. (J. org. Synth. 33 (1968), 4288) by cycloalkylating phenylacetic acid chlorides with ethylene, in the presence of aluminium chloride.

So far, said process has never been described for 2-arylpropionic acids.

It has now been found that cycloalkylation of 2-phenylpropionic acid (hydratropic acid) with ethylene in the presence of aluminium chloride makes possible the preparation of 1-methyl-2-tetralone in a single step and with good yields.

The present invention relates to the preparation of Nepinalone starting from 2-phenylpropionic acid chloride (II) and ethylene to give 1-methyl-2-tetralone (III), which is condensed with 1-N-(2-chloroethyl)piperidine (IV), and subsequently salified with HCl, according to the following scheme:

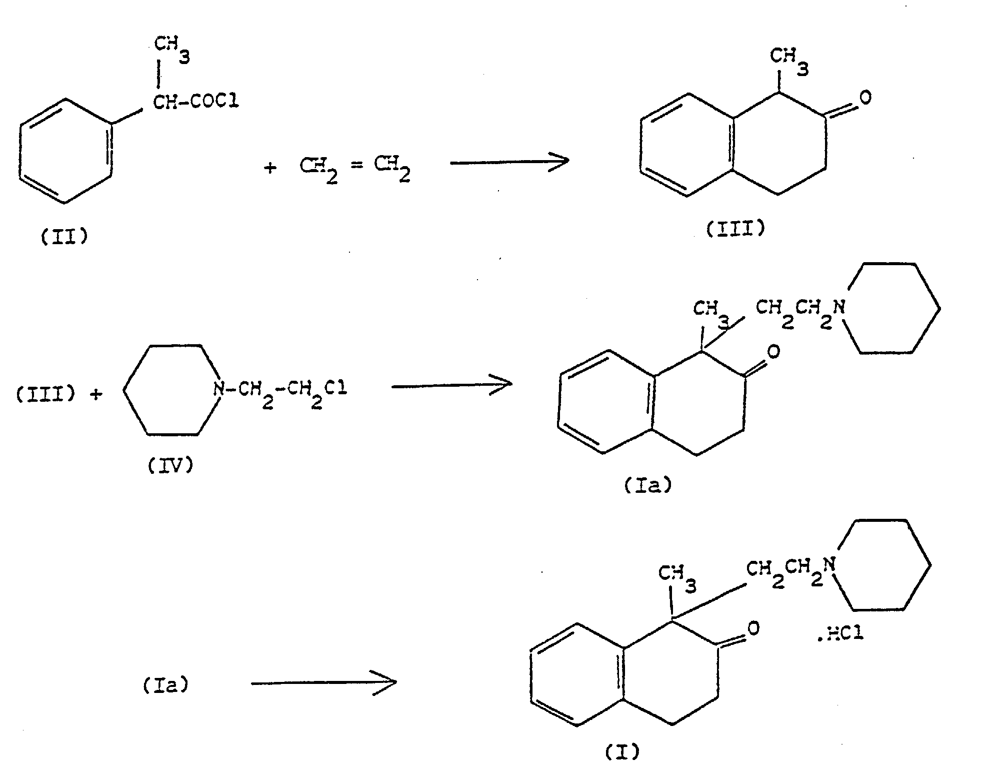

Said synthesis is advantageous when compared to the one described in GB 1.140.990, since 1-methyl-2-tetralone is obtained in a single step and in a good yield. Further, the final condensation between 1-methyl-2-tetralone (III) and 1-N-(2-chloroethyl)piperidine (IV) is carried out in an aliphatic hydrocarbon solvent, instead than an aromatic one. Said aspect is advantageous from the ecologic point of view, as liquid lower aliphatic hydrocarbons are less toxic and less pollutants.

The cyclization reaction, as carried out according to Burkhalter and Campbell (J. Org. Chem. 26, 4232 (1961)), has been optimized as per the starting acid chloride and the catalyst concentrations, which are critical to the yield of the final product.

Solvent amount may range from 2 to 4 l/mole of catalyst, preferably 3.6 l/mole, and from 4 to 7 1/mole of 2-phenylpropionic acid chloride, preferably 6 1/mole .

The process according to the present invention is characterized in that the reaction of 2-phenylpropionic acid chloride with ethylene is carried out in the presence of aluminium trichloride in a molar amount of about 0.44 mole per mole of 2-phenyl propionic acid chloride, at a temperature of -5°/-10° C, further cooling to -20°/-25° C, and in that a subsequent amount of about 0.96 mole of aluminium trichloride per mole of 2-phenyl propionic acid chloride is added and in that the temperature at the end of said subsequent amount is about -18°C.

Stirring enhances ethylene absorption, therefore reaction rate and yield. Halogenated hydrocarbons are used as solvent, among them CH₂Cl₂ is preferred.

The resulting 1-methyl-2-tetralone (III) is reacted with 1-N-(2-chloroethyl)piperidine (IV), through carbon anion. The reaction is carried out in the presence of an alkali metal, an hydride or amide thereof, in a lower aliphatic hydrocarbon as the solvent.

Sodium or potassium hydride, preferably sodium hydride dispersed in mineral oil, can be the alkali metal hydride.

The following examples further illustrate the invention.

### EXAMPLE 1

### 1-Methyl-1,2,3,4-tetrahydronaphthalen-2-one

100 g of 2-phenylpropionic acid chloride, dissolved in 1200 ml of CH₂Cl₂, were slowly dropped, in about 35-45 minutes, into a suspension containing 39 g of AlCl₃ in 1200 ml of CH₂Cl₂, cooled with brine (ethylene glycol stabilized in 50% aqueous solution wherein liquid nitrogen was bubbled) at -5°/-10°C. At the end of the addition, the suspension was further cooled to -20°/-25°C and gaseous ethylene was slowly blown, by means of a suitable device, for about 30 minutes, while vigorously stirring. After this time, the temperature in the reaction vessel was about -20°C. After 30 minutes at constant temperature, a mixture consisting of 85 g of AlCl₃ and 1200 ml of CH₂Cl₂ was added within 90 minutes. At the end of this time, the final temperature was about -18°C. Cooling was stopped, and after 90 minutes under vigorous stirring, the temperature into the vessel was -8°C. The brine was drained and the temperature was gradually let raise up to +5°C, in about 60 minutes, during which a low evolution of gaseous hydrochloric acid was observed. After cooling to 0°C, a 5% hydrochloric acid aqueous solution was dropped, within about 90 minutes. A vigorous heating reaction was observed. After standing overnight, the organic phase was separated from the aqueous one, which was washed with a small volume of methylene chloride. The organic phase was washed with a small portion of 5% HCl aqueous solution, then with a NaHCO₃ saturated solution and finally with distilled H₂O to reach about pH 7. The organic solution was dried over anhydrous sodium sulfate and evaporated to dryness. 100.5 g of a dark reddish oily residue were distilled in high vacuum (oil pump). 65 g of an oil (e.p. 93-95°; p = 2 mm Hg (267 Pa)) were obtained (yield 70%).

### EXAMPLE 2

### 1-Methyl-1-(β-piperidinoethyl)-1,2,3,4-tetrahydronaphthalen-2-one hydrochloride

In a 2 l vessel, 24 g of NaH suspension in 80% mineral oil, previously washed in heptane, were suspended in 300 ml of anhydrous heptane. The mixture was cooled down to -5°C and 100 g of 1-methyl-2-tetralone, dissolved in 400 ml of anhydrous heptane, were quickly added. At the end of the addition, the mixture was allowed to reach room temperature for about 1 hour; then the mixture was heated to 65-70°C for 3 hours. The reaction mixture was cooled again down to -10/-15°C and a solution containing 120 g of N-(2-chloroethyl)piperidine, which was recently freed from the hydrochloride thereof, in 800 ml of anhydrous heptane, was dropped very slowly about 80 minutes. At the end of the addition, the mixture was allowed to reach room temperature for about 40 minutes and then heated to 70°C for 3 hours. After standing overnight, the organic solution was washed several times with distilled water, then with a 10% w/v HCl aqueous solution. The acidic aqueous phase was strongly alkalized with concentrated sodium hydroxide and extracted with diisopropyl ether, washed with distilled water, dried over anhydrous sodium sulfate and evaporated to dryness. 160 g of an oily residue were obtained. After distillation with a high-vacuum pump, 120 g of oil (T = 140-145°C; p = 2 mm Hg (267 Pa)) were recovered. The distilled oil was purified through column chromatography. The column was packed with silica gel Merck 100 70-230 mesh; hexane was used as eluent. After discarding a first fraction containing impurities, the following fractions were collected, while monitoring by means of TLC, till about 80% of the pure oily product was eluted (about 96 g). Said oil was dissolved in ethyl acetate and precipitated in the form of hydrochloride with isopropanol/HCl 20% w/w. After filtration, 100.8 g of Nepinalone hydrochloride were recovered. Finally, the precipitate was recrystallized from a isopropyl ether/isopropanol mixture. After cooling, 91 g of final product were obtained (yield: 48%).

### EXAMPLE 3

### 1-Methyl-1-(β-piperidinoethyl)-1,2,3,4-tetrahydronaphthalen-2-one hydrochloride

In a 250 ml vessel, 7 g of unwashed NaH 80% dispersion in mineral oil were suspended in 30 ml of DMF and the suspension was cooled to -5°C; then 25 g of 1-methyl-2-tetralone, dissolved in 25 ml of DMF, were dropped rather quickly. A vigorous heating reaction was observed; at the end of the addition, the reaction mixture was heated up to 70°-80°C for about 2 hours, then the temperature was let go down to room temperature and the mixture was cooled to -10°C. 33 g of N-(β-chloroethyl)piperidine, in the form of free base, dissolved in 80 ml of anhydrous heptane, were dropped therein. At the end of the addition, the temperature was let to raise to the room one, then the reaction mixture was heated to 70°-80°C for 3 hours. After standing overnight, the suspension was poured into water and heptane separated from the DMF/H₂O mixture. After separating the phases, the aqueous phase was extracted twice with heptane. The combined organic extracts were washed several times with water, which showed strong alkalinity (pH > 10). The heptane phase was extracted with 1N HCl aqueous solution. The turbid acid extracts were reextracted with small portions of heptane; the acid liquors were alkalized and extracted with diisopropyl ether. The combined extracts were dried over sodium sulfate and evaporated to dryness. After distillation under high vacuum, 22.4 g of an oil (T = 120-130°C; p = 2 mm Hg (267 Pa)) were collected, with 60% final yield. 1-Methyl-(β-piperidinoethyl)-1,2,3,4-tetrahydronaphthalen-2-one was salified according to the method described in example 2.

## Claims

1. A process for the preparation of 1-methyl-1-[2-(1-N-piperidine)ethyl]-1,2,3,4-tetrahydronaphthalen-2-one hydrochloride (Nepinalone) of formula (I) 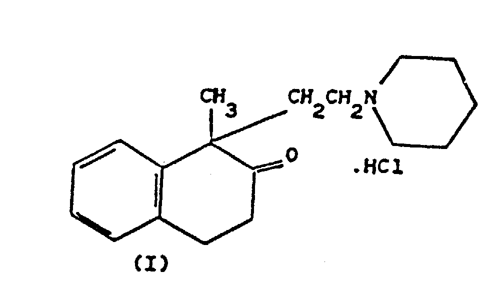 wherein 2-phenylpropionic acid, previously converted to the acid chloride thereof, is reacted with gaseous ethylene in the presence of aluminium trichloride at low temperature in an inert solvent, to give 1-methyl-1,2,3,4-tetrahydronaphthalen-2-one which is reacted with 1-N-(2-chloroethyl)piperidine, then salified with hydrochloric acid; characterized in that the reaction of 2-phenylpropionic acid chloride with ethylene is carried out in the presence of aluminium trichloride in a molar amount of about 0.44 mole per mole of 2-phenyl propionic acid chloride, at a temperature of -5°/-10° C, further cooling to -20°/-25° C, and in that a subsequent amount of about 0.96 mole of aluminium trichloride per mole of 2-phenyl propionic acid chloride is added and in that the temperature at the end of said subsequent amount is about -18°C.

2. The process according to claim 1, characterized in that methylene chloride is the solvent.

3. The process according to claim 1, characterized in that 1-methyl-1,2,3,4-tetrahydronaphthalen-2-one is reacted with N-(2-chloroethyl)piperidine in the presence of sodium hydride and an aliphatic hydrocarbon as the solvent.

4. The process according to claim 3, characterized in that n-heptane is the solvent.

## Patentansprüche

1. Verfahren zur Herstellung von 1-Methyl-1-[2-(1-N-piperidin)ethyl]-1,2,3,4-tetrahydronaphthalin-2-on-hydrochlorid (Nepinalon) der Formel (I) 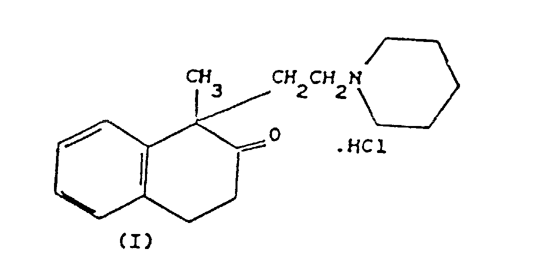 durch Umsetzung von 2-Phenylpropionsäure, die vorab in ihr Säurechlorid überführt wurde, mit gasförmigem Ethylen in Anwesenheit von Aluminiumtrichlorid bei niedriger Temperatur in einem inerten Lösungsmittel unter Bildung von 1-Methyl-1,2,3,4-tetrahydronaphthalin-2-on, welches mit 1-N-(2-Chlorethyl)piperidin umgesetzt, dann mit Chlorwasserstoffsäure in Salzform überführt wird, dadurch **gekennzeichnet,** daß die Reaktion von 2-Phenylpropionsäurechlorid mit Ethylen in Anwesenheit von Aluminiumtrichlorid in einer molaren Menge von etwa 0,44 Mol pro Mol 2-Phenylpropionsäurechlorid bei einer Temperatur von -5°/-10°C, weiterem Kühlen auf -20°/-25°C durchgeführt wird und daß eine darauffolgende Menge von etwa 0,96 Mol Aluminiumtrichlorid pro Mol 2-Phenylpropionsäurechlorid zugegeben wird und die Temperatur gegen Ende der darauffolgenden Menge etwa -18°C beträgt.

2. Verfahren nach Anspruch 1, dadurch **gekennzeichnet,** daß Methylenchlorid als Lösungsmittel verwendet wird.

3. Verfahren nach Anspruch 1, dadurch **gekennzeichnet,** daß 1-Methyl-1,2,3,4-tetrahydronaphthalin-2-on mit N-(2-Chlorethyl)piperidin in Anwesenheit von Natriumhydrid und einem aliphatischen Kohlenwasserstoff als Lösungsmittel umgesetzt wird.

4. Verfahren nach Anspruch 3, dadurch **gekennzeichnet,** daß N-Heptan als Lösungsmittel verwendet wird.

## Revendications

1. Un procédé de préparation du chlorhydrate de 1-méthyl-1-[2-(1-N-pipéridine)éthyl]-1,2,3,4-tétrahydronaphtalène-2-one (Népinalone) de formule (I) 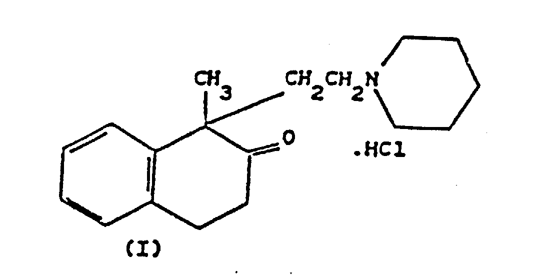 dans lequel on fait réagir de l'acide 2-phénylpropionique, préalablement converti en son chlorure d'acide, avec de l'éthylène gazeux en présence de trichlorure d'aluminium à basse température dans un solvant inerte, pour obtenir de la 1-méthyl-1,2,3,4-tétrahydronaphtalène-2-one que l'on fait réagir avec de la 1-N-(2-chloroéthyl)pipéridine, puis on salifie ensuite avec de l'acide chlorhydrique; caractérisé par le fait que la réaction du chlorure d'acide 2-phénylpropionique avec l'éthylène est réalisée en présence de trichlorure d'aluminium dans une proportion molaire d'environ 0,44 mole par mole de chlorure d'acide 2-phénylpropionique, à une température de -5°/-10°C, abaissée ensuite à -20°/-25°C, et par le fait que l'on ajoute ensuite une quantité d'environ 0,96 mole de trichlorure d'aluminium par mole de chlorure d'acide 2-phénylpropionique et que la température du milieu à la fin de cet ajout est d'environ -18°C.

2. Le procédé, conforme à la revendication 1, caractérisé par le fait que le solvant est du chlorure de méthylène.

3. Le procédé, conforme à la revendication 1, caractérisé par le fait que l'on fait réagir la 1-méthyl-1,2,3,4-tétrahydronaphtalène-2-one avec la N- (2-chloroéthyl)pipéridine en présence d'hydrure de sodium et d'un hydrocarbure aliphatique comme solvant.

4. Le procédé, conforme à la revendication 3, caractérisé par le fait que le solvant est le n-heptane.
